# EUROPEAN PATENT SPECIFICATION

(11) **EP 0 814 657 B1**
(45) Date of publication and mention of the grant of the patent: **26.05.1999**
(21) Application number: 96908065.4
(22) Date of filing: 19.03.1996
(51) Int. Cl.: A01M 1/20, A61L 9/03

(54) **SAFETY HEATER FOR LONG-LASTING INSECTICIDE OR DEODORANT MATS**
SICHERHEITS-HEIZVORRICHTUNG FÜR LANGHALTENDE MIT INSEKTIZIDEN ODER DEODORANT IMPRÄGNIERTE MATTEN
APPAREIL DE CHAUFFAGE DE SECURITE POUR MATS INSECTICIDES OU DEODORANTS DE LONGUE DUREE

(30) Priority: 21.03.1995 IT MI950560
(43) Date of publication of application: 07.01.1998
(73) Proprietor: ZOBELE INDUSTRIE CHIMICHE S.p.A., I-38100 Trento (IT); Zobele, Franco, 38100 Trento (IT); Pedrotti, Andrea, 30070 Pietramurata (IT)
(72) Inventor: ZOBELE, Franco, I-38100 Trento (IT); PEDROTTI, Andrea, I-30070 Pietramurata (IT)
(74) Representative: Faggioni, Giovanmaria, Dr.
(86) International application number: EP9601163
(87) International publication number: WO9628970

(56) References cited:
- EP-A- 0 671 123
- WO-A-91/17639
- DE-U- 8 907 992
- US-A- 4 257 558
- US-A- 4 795 883
- US-A- 5 378 254
- DATABASE WPI Week 8747 Derwent Publications Ltd., London, GB; AN 87-331247 XP002004501 & JP,A,62 236 551 (ENKLAR BUSINESS KK)

## Description

The present invention concerns a safety heater for long-lasting mats and, in particular, a heater to exhale insecticide or deodorant substances.

The use of heaters to spread insecticides into closed (or even just partially closed) environments, has been adopted since many years. Said use has also been extended to mats for exhaling deodorants, with a principle similar to that adopted for insecticides.

Quite recently, the Applicant has advantageously introduced, with the EP-A1-0 671 123, the use of plastic mats to replace the traditional cardboard ones. This new type of mat, consisting of a block copolymer formed by an elastomeric polymer and a stiff polymer, allows a more gradual spreading of the active ingredient, a far longer action of the filling charge - lasting, for instance, up to 30-45 nights - and, consequently, a considerable financial saving and further convenience in use.

A fundamental task of the heater according to the present invention is to heat this new type of mat, so as to cause the vaporisation of the active ingredient contained therein. Moreover, since these new long-lasting mats have, as seen, a useful life of several weeks, it is practically indispensable for the heater to be able to supply an indication as to the period or use of the mat, so as to reckon exactly when said mat is really exhausted. This problem is specially felt, when using the heater only occasionally. Finally since, as known, the heater can get very hot during use, some of its inner components may become quite dangerous upon contact - in case they should be accidentally disassembled - especially for children. In particular, the user could easily get burnt then trying to replace an exhausted mat by a new one, while the heater is working.

Such problems and drawbacks are now brilliantly solved and overcome by the present invention which, according to a first embodiment thereof, provides a safety heater for long lasting insecticide or deodorant mats, of a type which shrinks as it lets out the insecticide or deodorant substance, characterised in that said safety heater comprises means for signalling the life period of said mat in the form of a mechanical indicator provided with at least one element which is in contact with the edge of the mat, and apt to detect and evidence the shrinkage undergone by the mat (4) during its use.

In a second embodiment of the present invention, is provided a safety heater for long lasting insecticide or deodorant mats, characterised in that it comprises means for signalling the life period of said mat in the form of a graduated toothed wheel moved by a pawl, and reference means, preferably in the form of a peephole, formed in the casing of the heater, and in that said pawl and said heater are controlled by common operating means.

Preferably, the heater of the present invention further comprises safety means which consist of a screening grid to cover the heating element of the heater, apt to withhold the mat and to be fixed to and removed from the heater by sliding exclusively in the direction of the plug feeding current to said heater. Advantageously, the sliding of said screening grid is controlled by a special release push-button.

The invention is moreover particularly effective if the heater is refilled with a plastic mat already fixed to said screening grid, so as to avoid direct contact with the hands.

The present invention will now be described in further detail, with reference to the accompanying drawings, which illustrate some preferred embodiments thereof and in which:
Fig. 1 is a plan view partially looking through a heater according to the present invention;
Fig. 2 is a partly sectional front view of the heater, along the line II-II of Fig. 1;
Fig. 3 is a partly sectional side view of the heater, along the line III-III of fig. 1;
Figs. 4a to 4c are three views of a switch of the heater according to the present invention;
Figs. 5a to 5c are three views of a plug feeding current to the heater of the present invention;
Fig. 6 is an assembly view of the device for signaling the life period of the mat and of the switch of the heater, according to a first embodiment of the present invention;
Figs. 7a-7b are plan views of the means for signaling the life period of the mat, respectively in a starting and final condition of use thereof, according to a second embodiment of the present invention;
Figs. 8a-8b are plan views of the means for signaling the life period of the mat: respectively in a starting and final condition of use thereof, according to a third embodiment of the present invention; and
Figs. 9a-9b are lateral section views of a heater: respectively in a starting and final condition of use of the mat, according to a fourth embodiment of the present invention.

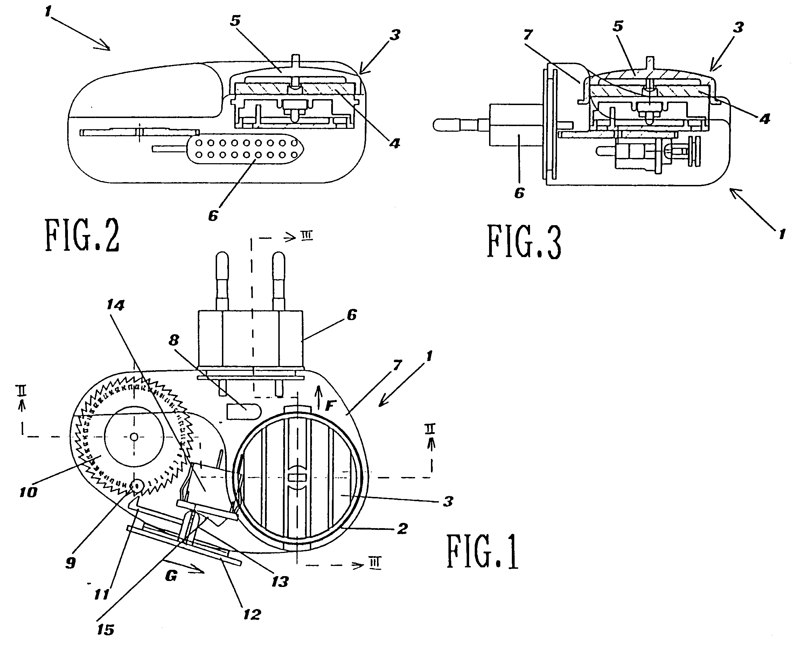

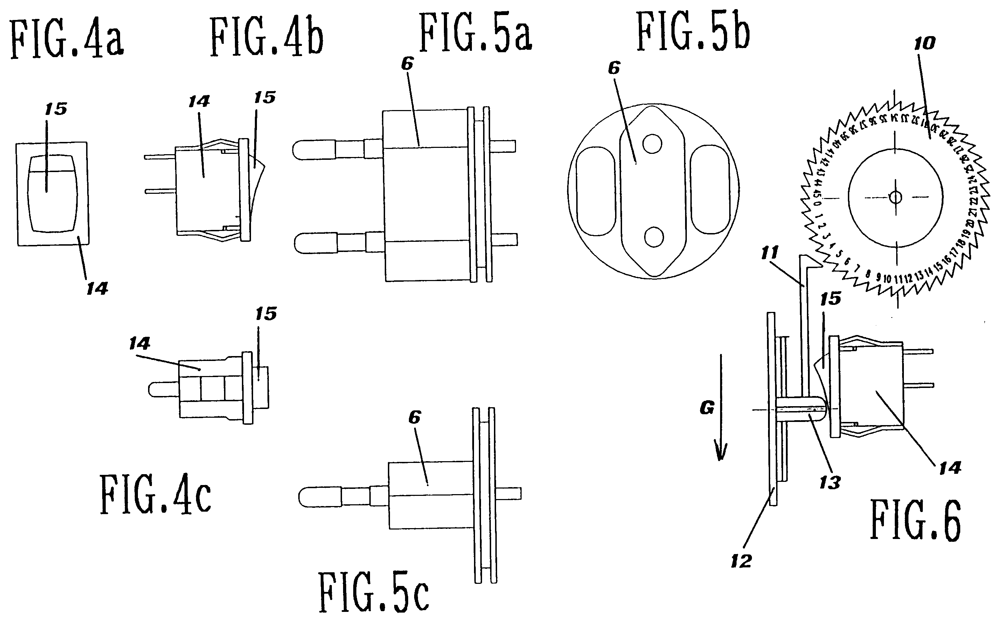

As shown in Figures 1 to 3, the heater 1 according to the present invention comprises a round seat 2, into which is inserted a refill 3, consisting of a mat 4 of plastic material and of a screening grid 5. The heater 1 is fed with current by a rotary plug 6, shown more clearly in figs. 5a to 5c. The refill 3 is removable from the heater 1 by sliding in respect of the wall 7 of its casing, exclusively in the direction of the plug 6, after having been released by means of a special push-button 8. The release button 8 should be pushed in a direction such as not to be naturally coordinate with the sliding direction F of the refill 3, and thus make it more unlikely for a child to accidentally cause the removal of said refill.

Through a peephole 9, provided on the casing wall 7 of the heater, it is possible to see one of the numbers reported on a special graduated toothed wheel 10, meant to indicate the number of days or nights of use, or preferably, the number of days or nights that are still available for use of the refill 3. The toothed wheel 10 is apt to be engaged by a pawl 11 controlled at its other end by a slider 12. In fact, a pin 13 projects inwards from said slider 12 and carries the pawl 11; said pin 13 also acts onto a switch 14, allowing to switch on the heater 1 by pressing its button 15, when the slider 12 is moved in the direction indicated by the arrow G. The switch 14 is shown in detail in figs. 4a to 4c and it is of a type normally available on the market, possibily also backlighted; in this last case: the wall 7 of the heater 1 comprises an at least partially transparent window in correspondence of said switch 14. Fig. 6 shows in detail the unit formed by the switch 14 and by the means for signaling the life period of the mat 4, which comprise the graduated toothed wheel 10, the pawl 11 and the slider 12.

As previously mentioned, among the long-lasting mats of plastic material, there are known to be mats which reduce in size as they let out the active ingredient during use; said shrinkage is directly proportional to the time of use, or life of the mat. Nevertheless, one cannot take direct advantage of this property, seen that said reduction in size is so gradual and unperceivable as not to be noticed at first sight. A description will be given hereinafter of some alternative embodiments which take advantage of this property, adopting means apt to place in evidence the progressive shrinkage of the mat by suitably emphasizing the same.

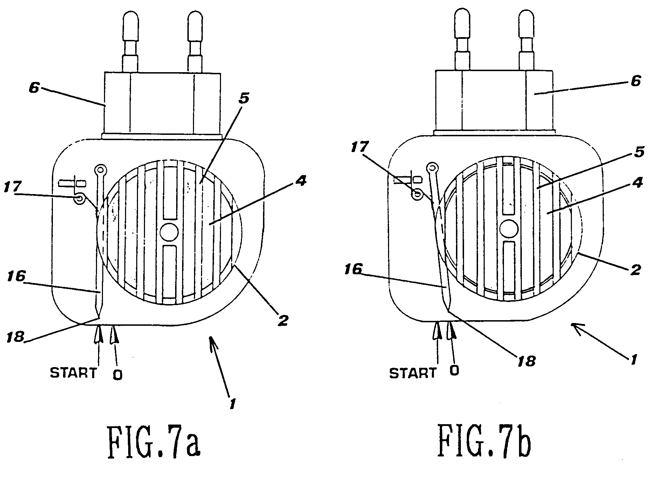

Figs. 7a and 7b show an alternative embodiment of the means for signaling the life period of the mat 4, which comprise in this case a pointer 16 pressed against the mat 4 by a spring 17, the tip 18 of said pointer 16 indicating a position between the start and end of life (START - 0) of the mat 4.

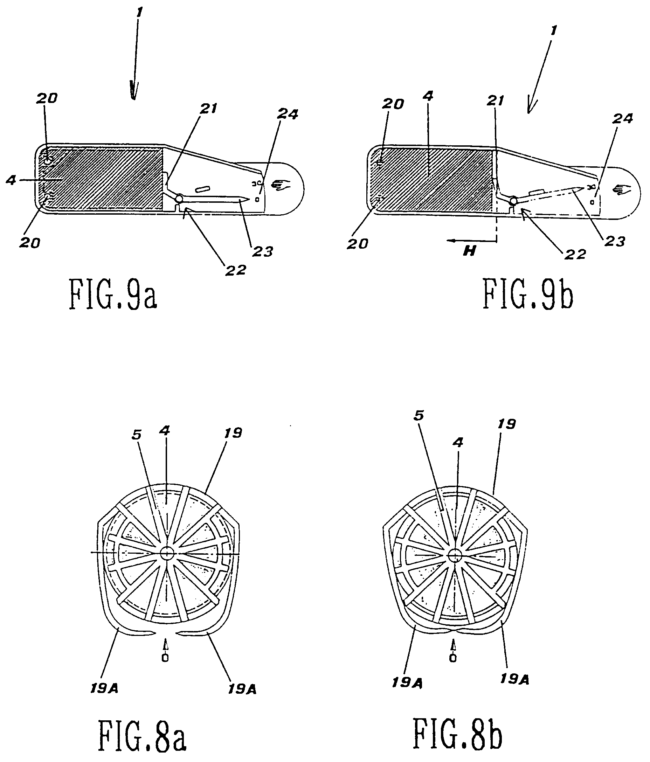

An even further embodiment of said signaling means is shown in figs. 8a and 8b, wherein the screening grid 5 apt to withhold the mat 4 has a seat 19 in the form of tongs of elastic material, conceived so that its arms 19A constantly adhere to the edge of said mat 4 and indicate, with their respective points, the gradual approach to the end of life (mark "0") of the mat 4.

Finally, the embodiment shown in figs. 9a and 9b foresees the use of a mat 4 having a rectangular shape substantially identical to that of conventional mats. In this case, an end of the mat 4 is held in position by fixing means 20, such as pins, while against its opposite free end there leans an arm 21 of a rocking lever 22, whose other arm 23 points towards a counter 24 showing the degree of use of the mat.

As seen, the heater 1 is used to release from the refill 3 a volatile substance, such as an insecticide, a deodorant, a scent or the like. Nevertheless such substances, with which the mat, 4 is imbued, may cause irritations or allergies once they are in contact with the hands; they can moreover transmit, by contact, part of the smell in a persistent way (especially when using deodorant mats) and can , anyhow, result sticky or damp to the touch. The arrangement of the refill 3 according to the present invention, comprising a mat 4 firmly held into a screening grid 5, thus turns out to be particularly advantageous, in that it prevents contact with the hands whenever having to replace said mat.

When using the heater 1 of the present invention, the plug 6 thereof has to be previously disconnected from the power socket to allow removing the refill 3. In fact, to remove the refill 3 from the casing of the heater, one should push the button 8 so as to release said refill and then cause it to slide in the direction of arrow F (fig. 1) towards the rotary plug 6. This arrangement and sliding direction prevent any accidental or voluntary displacement of the refill 3, when the plug is inserted, hence eliminating all risks of burns when the heater 1 is switched on.

After having positioned a new refill 3 in its seat 2, said refill is automatically blocked into the heater 1 by tripping of the button 8. Said heater, thus equipped with a plastic mat 4, is generally switched on at night time, in the case of an insecticide, and in the morning when a deodorant is required. The confection of the refill 3 normally reports the number of nights or of days of its lasting period. In the case of the first embodiment of the invention, one therefore rotates by hand the graduated toothed wheel 10, until the above reported number of nights, or of days, is positioned so as to be read through the peephole 9. At this point: the rotary plug 6 can be inserted into the power socket, which can be either vertical or horizontal, since the casing of the heater 1 can be shifted into the required position thanks to the rotation of said plug 6.

To operate the heater 1, the slider 12 is moved in the direction of the arrow G (figs. 1 and 6); in this way, the pin 13 causes the button 15 to close the switch 14, thereby operating the heater 1 which heats the mat 4. Simultaneously, the pin 13 draws along also the pawl 11, which engages a tooth of the wheel 10, causing it to shift by one position and thus place in evidence, through the peephole 9, the number just below the previous one.

To stop the heater from working, the slider 12 is moved back into its former position, thereby opening again the switch 14 through the button 15. The pin 13 also moves back again the pawl 11, which thus engages another tooth. In this way, it is always possible to read through the peephole 9 the number of days, or of nights, that are still available before exhaustion of the refill 3. In fact, when the number "0" appears, it is time to replace the refill 3 and to again proceed as described heretofore.

According to the embodiment illustrated in figs. 7a and 7b, the new refill 3 is inserted into the seat 2 making sure that the pointer 16 is pressed by the spring 17 against an outer edge of the mat 4. As soon as the refill 3 has been inserted, the tip 18 of the pointer 16 indicates the position of "START" (fig. 7a). As the active ingredient wears out, the mat 4 substantially reduces its size, shrinking in respect of its central fixing point to the screening grid 5. Thanks to the action of the spring 17, the pointer 16 always keeps in contact with the edge of the mat 4, so that, as the active ingredient wears out, the tip 18 of said pointer gradually shifts towards the position "0", which it finally reaches (fig. 7b) upon exhaustion of the active ingredient. At this point, the refill 3 is replaced by a new one in the manner specified heretofore.

In the embodiment of figs. 8a and 8b, a new refill 3 comprises a screening grid 5, with arms 19A which surround the mat 4 - elastically adhering thereto - in such a way (fig. 8a) as to keep their ends parted. Thanks to the elasticity of the arms 19A, they always adhere onto the mat 4 during use, whereby, as said mat gradually shrinks, the ends of said arms draw closer and closer until, upon exhaustion of the mat 4, they touch each other in correspondence of "0" (fig. 8b). At this point, the refill 3 needs to be replaced.

The mat 4, shown in figs. 9a and 9b, has the same shape as the conventional mats and it can be heated through a remote source, by convection or radiance, at temperatures higher than usual. Besides, the advantage of said mat is that it can be used with a known-type heater. When said mat is preferably used in the heater 1 of figs. 9a and 9b, it is previously fixed into said heater, by one of its ends, with a pair of pins 20. The arm 21 of the rocking lever 22 must be previously shifted by hand, to allow inserting the mat 4 with its free end bearing against said arm 21. In this way, the other arm 23 of said rocking lever 22 is led to point towards the mark "0" shown on the counter 24 (fig. 9a). During use, the mat 4 shrinks, but the presence of the pins 20 produces the shrinkage only in correspondence of its free end, namely in the direction indicated by the arrow H. The arm 21 thus gradually moves and, with it, the arm 23 which hence points time after time, at the number of the days of use reported on the counter 24. When the arm 23 points at the final number of days of use (for instance 30, in the particular embodiment shown in fig. 9b), one draws out the mat 4 so as to replace it by a new one. For the sake of clearness, the counter 24 shown in figs. 9a and 9b reports only the numbers "0" and "30", but said counter may obviously report more fiducial marks, even one for each days of use.

As can be understood from the previous description, the use of a heater according to the present invention is quite simple and obvious, while being extremely safe in that it considerably reduces the risk of accidents. Moreover, thanks to the presence of the means for signaling the life period of the mat, it allows to fully use up the refill 3, with no wastes nor an ineffective use thereof.

The present intention has been described with reference to some preferred embodiments thereof, but it is understood that any variants and additions can be introduced without thereby departing from its scope. For instance, in the first embodiment of the means for signaling the life period of the mat 4, the graduated toothed wheel 10 - when rotating - instead of reporting the number of days or of nights of use in a decreasing clockwise direction (i.e. from 45 to 0), can equally well report said number of days or nights in an increasing clockwise direction, so that the number of days or nights appearing through the peephole 9 will correspond to that of use of said mat.

## Claims

1. Safety heater for long lasting insecticide or deodorant mats (4), of a type which shrinks as it lets out the insecticide or deodorant substance, characterized in that said safety heater comprises means for signaling the life period of said mat (16, 17, 18; 19A; 21, 22, 23, 24) in the form of a mechanical indicator provided with at least one element which is in contact with the edge of the mat, and apt to detect and evidence the shrinkage undergone by the mat (4) during its use.

2. Heater as in claim 1), wherein said mechanical indicator consists of a pointer (16), pivoted at one end and a side of which is pressed by elastic means (17) against and edge of said mat (4), and of fiducial marks (START - 0), indicating the start and end of life of the mat, reported onto the casing of the heater (1) close to the tip (18) of said pointer (16).

3. Heater as in claim 1), wherein said mechanical indicator consists of two arms (19A) acting as tongs, elastically adhering to opposite edges of the mat (4) and forming, with their ends, pointers to indicate the life period of said mat (4).

4. Heater as in claim 3), wherein said mechanical indicator also comprises fiducial marks indicating the start and end of life of the mat, reported onto the casing of the heater (1) close to the tips of said pointers.

5. Heater as in claim 3), wherein the tips of said pointers are apt to contact each other when the life of said mat (4) has come to an end.

6. Heater as in claim 1), wherein said mechanical indicator consists of a rocking lever (22), an arm (21) of which leans against an edge of the mat (4) and the other arm (23) of which points at a counter (24) reporting fiducial marks indicating the start and end of life of the mat.

7. Heater as in claim 6), wherein said mat (4) is fixed to the heater (1) at its end opposite to that contacting said rocking lever (22).

8. Safety heater for long lasting insecticide or deodorant mats (4), characterized in that it comprises means for signaling the life period of said mat (9, 10, 11) in the form of a graduated toothed wheel (10) moved by a pawl (11), and reference means, preferably in the form of a peephole (9), formed in the casing of the heater (1), and in that said pawl (10) and said heater (1) are controlled by common operating means (12, 13).

9. Heater as in claim 8), wherein said common operating means comprise a hand-operatable slider (12), from which a pin (13) projects towards the inside of the heater (1), said pin (13) carrying said pawl (11) and engaging with a switch (14) of the heater (1).

10. Heater as in claim 8) or 9), wherein the graduation reported on said toothed wheel (10) comprises numbers arranged in a decreasing clockwise direction.

11. Heater as in claim 8) or 9), wherein the graduation reported on said toothed wheel (10) comprises numbers arranged in an increasing clockwise direction.

12. Safety heater as in any one of the preceding claims, further comprising safety means (3, 8) apt to prevent the replacement or accidental removal of the mat (4) while the heater (1) is working.

13. Heater as in claim 12), wherein said safety means comprise a refill (3), consisting of a mat (4) and of a screening grid (5) apt to cover the heating element of the heater and to withhold said mat (4), said refill (3) being fixable to and removable from the heater (1) by sliding exclusively in the direction of the plug (6) feeding current to said heater.

14. Heater as in claim 13), wherein said safety means also comprise a release button (8) to control the sliding of said refill (3).

## Patentansprüche

1. Sicherheits-Heizvorrichtung für langhaltende mit Insektiziden oder Deodorant imprägnierte Matten (4) von der Art, die bei Herauslassen des Insektizids oder Deodorants schrumpft, dadurch gekennzeichnet, daß die Sicherheits-Heizvorrichtung ein Mittel zum Signalisieren der Lebensdauer der Matte (16, 17, 18; 19A; 21, 22, 23, 24) in Form einer mechanischen Anzeigeeinrichtung umfaßt, die mit wenigstens einem Element versehen ist, das sich in Kontakt mit der Kante der Matte befindet, und das zum Detektieren und Nachweisen des von der Matte (4) während des Gebrauchs durchgemachten Schrumpfens fähig ist.

2. Heizvorrichtung nach Anspruch 1, worin die mechanische Anzeigeeinrichtung aus einem Zeiger (16), der an einem Ende drehbar gelagert ist und dessen eine Seite durch ein Federmittel (17) gegen eine Kante der Matte (4) gedrückt ist, und aus Bezugspunkten (START - 0) besteht, die den Beginn und das Ende der Lebensdauer der Matte anzeigen und auf dem Gehäuse der Heizvorrichtung (1) dicht bei der Spitze (18) des Zeigers (16) angegeben sind.

3. Heizvorrichtung nach Anspruch 1, worin die mechanische Anzeigeeinrichtung aus zwei Armen (19A) besteht, die als elastisch an gegenüberliegenden Kanten der Matte (4) hängende Zungen wirken und mit deren Enden Zeiger zum Anzeigen der Lebensdauer der Matte (4) bilden.

4. Heizvorrichtung nach Anspruch 3, worin die mechanische Anzeigeeinrichtung auch Bezugspunkte umfaßt, die den Beginn und das Ende der Lebensdauer der Matte anzeigen und auf dem Gehäuse der Heizvorrichtung (1) dicht bei den Spitzen der Zeiger angegeben sind.

5. Heizvorrichtung nach Anspruch 3, worin die Spitzen der Zeiger zum Kontakt miteinander fähig sind, wenn die Lebensdauer der Matte (4) sich zum Ende neigt.

6. Heizvorrichtung nach Anspruch 1, worin die mechanische Anzeigeeinrichtung aus einem Schwenkhebel (22) besteht, dessen einer Arm (21) gegen eine Kante der Matte (4) lehnt und dessen anderer Arm (23) auf eine Zähleinrichtung (24) zeigt, die den Beginn und das Ende der Lebensdauer der Matte anzeigende Bezugspunkte angibt.

7. Heizvorrichtung nach Anspruch 6, worin die Matte (4) an ihrem Ende gegenüber dem den Schwenkhebel (22) berührenden an der Heizvorrichtung (1) befestigt ist.

8. Sicherheits-Heizvorrichtung für langhaltende mit Insektiziden oder Deodorant imprägnierte Matten (4), dadurch gekennzeichnet, daß sie ein Mittel zum Signalisieren der Lebensdauer der Matte (9, 10, 11) in Form eines von einer Sperrklinke (11) bewegten Meßzahnrades (10) und eine Sichteinrichtung, vorzugsweise in Form eines Guckloches (9), das in dem Gehäuse der Heizvorrichtung (1) ausgebildet ist, umfaßt und daß die Schaltklinke (10) und die Heizvorrichtung (1) von einem gemeinsamen Betätigungsmittel (12, 13) gesteuert werden.

9. Heizvorrichtung nach Anspruch 8, worin das gemeinsame Betätigungsmittel einen mit der Hand betätigbaren Gleitschieber (12) umfaßt, von dem ein Stift (13) zu der Innenseite der Heizvorrichtung (1) vorragt, wobei der Stift (13) die Schaltklinke (11) trägt und mit einem Schalter (14) der Heizvorrichtung (1) in Eingriff steht.

10. Herzvorrichtung nach Anspruch 8 oder 9, worin die auf dem Zahnrad (10) angegebene Unterteilung Nummern umfaßt, die im Uhrzeigersinn in absteigender Form angeordnet sind.

11. Heizvorrichtung nach Anspruch 8 oder 9, worin die auf dem Zahnrad (10) angegebene Unterteilung Nummern umfaßt, die im Uhrzeigersinn in ansteigender Form angeordnet sind.

12. Sicherheits-Heizvorrichtung nach irgendeinem der vorangehenden Ansprüche, zusätzlich mit einem Sicherheitsmittel (3, 8), das zum Verhindern des Auswechselns oder zufälligen Entfernens der Matte (4) während des Betriebs der Heizvorrichtung (1) fähig ist.

13. Heizvorrichtung Anspruch 12, worin das Sicherheitsmittel eine Nachfüllpackung (3) umfaßt, die aus einer Matte (4) und aus einem Abschirmgitter (5) besteht, das zum Abdecken des Heizelements der Heizvorrichtung und zum Zurückhalten der Matte (4) fähig ist, wobei die Nachfüllpackung (3) an der Heizvorrichtung (1) befestigbar und durch Schieben ausschließlich in die Richtung des Stroms zu der Heizvorrichtung speisenden Steckers (6) aus der Heizvorrichtung (1) entfernbar ist.

14. Heizvorrichtung nach Anspruch 13, worin das Sicherheitsmittel auch einen Freigabeknopf (8) zum Steuern des Gleitens der Nachfüllpackung (3) umfaßt.

## Revendications

1. Dispositif chauffant à sécurité pour plaquettes d'insecticide ou de désodorisant à longue durée (4), d'un type qui rétrécit au fur et à mesure de la diffusion de la substance insecticide ou désodorisante, caractérisé en ce que ledit dispositif chauffant à sécurité comprend un moyen (16, 17, 18 ; 19A; 21, 22, 23, 24) pour signaler la durée de vie de ladite plaquette sous la forme d'un indicateur mécanique doté d'au moins un élément qui est en contact avec le bord de la plaquette, et adapté à détecter et mettre en évidence le rétrécissement subi par la plaquette (4) durant son utilisation.

2. Dispositif chauffant selon la revendication 1, dans lequel ledit indicateur mécanique consiste en une aiguille (16), pivotant à une extrémité et dont un côté est pressé par un moyen élastique (17) contre un bord de ladite plaquette (4), et en marques de repère (DEBUT - 0), indiquant le début et la fin de la vie de la plaquette, signalées sur le boîtier du dispositif chauffant (1) près de la pointe (18) de ladite aiguille (16).

3. Dispositif chauffant selon la revendication 1, dans lequel ledit indicateur mécanique consiste en deux bras (19A) servant de pinces, adhérant élastiquement aux bords opposés de la plaquette (4) et formant, avec leurs extrémités, des aiguilles pour indiquer la durée de vie de ladite plaquette (4).

4. Dispositif chauffant selon la revendication 3, dans lequel ledit indicateur mécanique comprend également des marques de repère indiquant le début et la fin de la vie de la plaquette, signalées sur le boîtier du dispositif chauffant (1) près des pointes desdites aiguilles.

5. Dispositif chauffant selon la revendication 3, dans lequel les pointes desdites aiguilles sont adaptées à se contacter lorsque la vie de ladite plaquette (4) se termine.

6. Dispositif chauffant selon la revendication 1, dans lequel ledit indicateur mécanique consiste en un levier oscillant (22), dont un bras (21) s'appuie contre un bord de la plaquette (4) et l'autre bras (23) pointe vers un compteur (24) doté de marques de repère indiquant le début et la fin de la vie de la plaquette.

7. Dispositif chauffant selon la revendication 6, dans lequel ladite plaquette (4) est fixée au dispositif chauffant (1) à son extrémité à l'opposé de celle en contact avec ledit levier oscillant (22).

8. Dispositif chauffant à sécurité pour plaquettes d' insecticide ou de désodorisant à longue durée (4), caractérisé en ce qu'il comprend un moyen (9, 10, 11) pour signaler la durée de vie de ladite plaquette sous la forme d'une roue dentée graduée (10) déplacée par un cliquet (11), et un moyen de référence, de préférence sous la forme d'une fenêtre (9), ménagée dans le boîtier du dispositif chauffant (1), et en ce que ledit cliquet (11) et ledit dispositif chauffant (1) sont commandés par un moyen d'actionnement commun (12, 13).

9. Dispositif chauffant selon la revendication 8, dans lequel ledit moyen d'actionnement commun comprend une pièce coulissante à commande manuelle (12), d'où une tige (13) se projette vers l'intérieur du dispositif chauffant (1), ladite tige (13) supportant ledit cliquet (11) et s'engageant avec un interrupteur (14) du dispositif chauffant (1).

10. Dispositif chauffant selon la revendication 8 ou 9, dans lequel la graduation signalée sur ladite roue dentée (10) comprend des nombres décroissant dans le sens des aiguilles d'une montre.

11. Dispositif chauffant selon la revendication 8 ou 9, dans lequel la graduation signalée sur ladite roue dentée (10) comprend des nombres croissant dans le sens des aiguilles d'une montre.

12. Dispositif chauffant à sécurité selon l'une quelconque des revendications précédentes, comprenant, en outre, un moyen de sécurité (3, 8) adapté à empêcher le remplacement ou l'enlèvement accidentel de la plaquette (4) tandis que le dispositif chauffant (1) fonctionne.

13. Dispositif chauffant selon la revendication 12, dans lequel ledit moyen de sécurité comprend une recharge (3), consistant en une plaquette (4) et en une grille-écran (5) adaptée à recouvrir l'élément de chauffage du dispositif chauffant et à retenir ladite plaquette (4), ladite recharge (3) étant adaptée à être fixée au, et enlevée du, dispositif chauffant (1) par glissement uniquement dans le sens de la fiche (6) alimentant ledit dispositif chauffant en courant.

14. Dispositif chauffant selon la revendication 13, dans lequel ledit moyen de sécurité comprend également un bouton de libération (8) pour commander le glissement de ladite recharge (3).
